# FASCICULE DE BREVET EUROPEEN

(11) **EP 1 129 667 B1**
(45) Date de publication et mention de la délivrance du brevet: **07.03.2007**
(21) Numéro de dépôt: 01810204.6
(22) Date de dépôt: 28.02.2001
(51) Int. Cl.: A61B 17/16

(54) **Porte-outil pour outil chirurgical**
Werkzeugträger für ein chirurgisches Instrument
Tool holder for a surgical instrument

(30) Priorité: 02.03.2000 CH 4092000
(43) Date de publication de la demande: 05.09.2001
(73) Titulaire: PRECIMED S.A., 2534 Orvin (CH)
(72) Inventeur: Lechot, André, 2534 Orvin (CH)
(74) Mandataire: Mötteli-Mantelli, Novella

(56) Documents cités:
- EP-A- 0 782 840
- FR-A- 2 744 629
- US-A- 5 817 096
- US-A- 5 976 144

## Description

L'invention a pour objet un porte-outil pour outil chirurgical comprenant un manche muni d'une tête conformée pour recevoir un outil et une pièce annulaire de verrouillage montée coulissante autour du manche, sous la tête, munie de moyens de verrouillage coopérant avec la tête pour verrouiller l'outil sur la tête et poussée contre la tête par un ressort hélicoïdal.

Un tel porte-outil est connu notamment du brevet européen 0 704 191 et du brevet US 5,236,433.

FR-A-2 744 629 décrit une instrumentation qui comprend une fraise 2, adaptable sur un porte-fraise 1 et conformée pour constituer par elle-même un cotyle d'essai permettant de recevoir une tête d'essai de prothèse fémorale. Le but de cette invention consiste à fournir une fraise 2 qui peut être fixée sur le bassin 34 par des clous 29, son orientation étant repérée par les moyens de visée 32 du porte-fraise 1. Il n'est pas envisagé de fournir un moyen de permettre la solidarisation/désolidarisation en vue du désassemblage facilité.

Un outil chirurgical, par exemple pour la préparation de la mise en place d'une prothèse de la hanche, travaille dans un milieu salissant fortement l'outil et le porte-outil. D'autre part, un porte-outil chirurgical doit être nettoyé très fréquemment et très soigneusement afin d'éviter tout risque d'infection. Or, le nettoyage des instruments chirurgicaux est difficile, en particulier le nettoyage de l'espace entre le manche et la pièce de verrouillage en raison de la présence de débris d'os et de sang coagulé.

L'invention a pour but d'assurer des conditions de nettoyage optimales et rapides.

A cet effet, le porte-outil selon l'invention est caractérisé en ce que le ressort de poussée s'appuie sur une bague coulissant sur le manche et en ce que le manche et la bague présentent des moyens de solidarisation, de telle manière que la libération de la bague autorise le libre coulissement de la pièce de verrouillage, du ressort et de la bague sur le manche.

Ce démontage quasi instantané des pièces composant le porte-outil permet son parfait nettoyage et ceci peut être fait très rapidement.

Selon un mode d'exécution préféré de l'invention, le manche présente, sous la tête, une section de diamètre supérieur au diamètre du reste du manche, section sur laquelle la bague vient se fixer par une fixation à baionnette.

Le jeu des composants des moyens de verrouillage sur le manche permet un bon nettoyage sans qu'il soit nécessaire d'enlever ces composants du manche, ce qui évite le risque de perdre une pièce ou de les mélanger et évite de devoir remonter les composants sur le manche. La fixation et la libération de la bague se font instantanément ce qui constitue un gain de temps. Ceci permet d'éviter de rendre inexploitable un kit complet d'instruments pour une seule pièce rendue inopérante.

La tête et les moyens de fixation et de verrouillage de l'outil peuvent être réalisés de nombreuses manières. Ces moyens ne font pas partie de l'invention proprement dite.

Le dessin annexé représente, à titre d'exemple, un mode d'exécution de l'invention.
La figure 1 représente le porte-outil en position démontée.
La figure 2 représente le porte-outil en position verrouillée.

Le porte-outil représenté comprend un manche cylindrique 1 à une extrémité duquel est fixée une tête 2 identique à la tête décrite dans le brevet EP 0 704 191 (US 5,658,290). Rappelons que cette tête présente une creusure centrale, la tête formant une couronne autour de cette creusure. Cette couronne présente quatre crans 3 à baïonnette diamétralement opposés par paire. Dans ces crans 3 vient se fixer une fraise analogue à la fraise représentée et décrite dans le brevet EP 0 704 191. La fraise et verrouillée dans les crans 3 par une pièce annulaire de verrouillage 4 munie d'un flasque 5 portant quatre doigts parallèles 6 traversant la tête 2 pour venir fermer les crans de baïonnette 3, comme décrit dans le brevet EP 0 704 191.

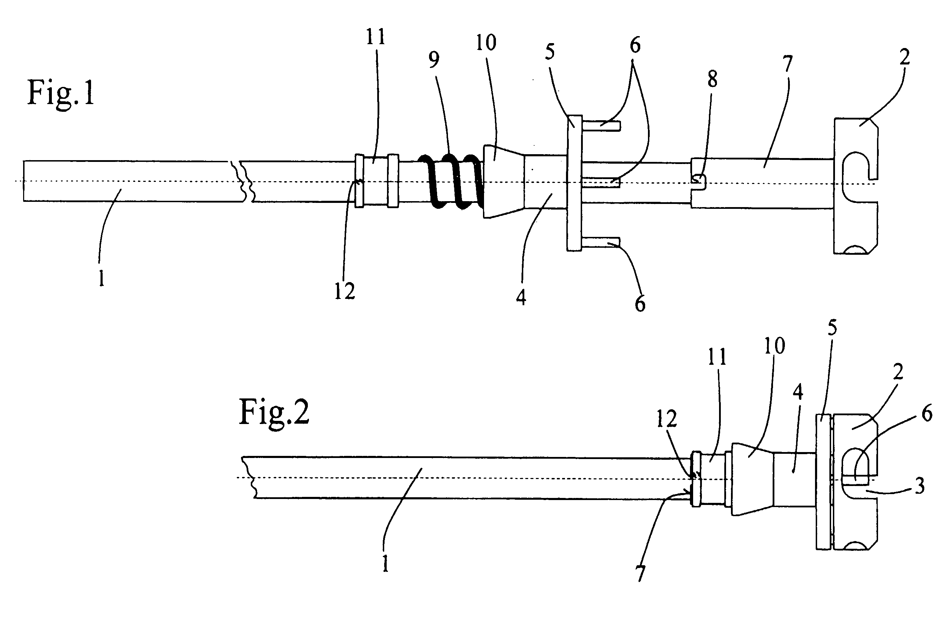

La pièce de verrouillage 4 ne coulisse pas directement sur la section du manche visible à la figure 1, mais sur une section 7 de diamètre supérieur au diamètre du reste du manche. Cette section 7 peut être constituée d'une pièce tubulaire chassée sur le manche 1. A l'extrémité de la section 7 opposée à la tête 2 est formé au moins un cran de baïonnette 8. Ces crans sont de préférence au moins au nombre de deux et diamétralement opposés pour faciliter l'assemblage comme on le verra plus loin. Autour de cette section 7 est en outre monté un ressort hélicoïdal 9 s'engageant dans une partie évasée tronconique 10 de la pièce de verrouillage 4 et venant s'appuyer contre cette pièce de verrouillage dont la partie médiane coulisse librement sur la section 7. Le porte-outil est complété par une bague 11 coulissant également sur la section 7 et munie intérieurement d'un ergot radial 12, c'est-à-dire dirigé en direction du manche 1.

A partir de la position démontée représentée à la figure 1, pour monter le porte-outil on amène la pièce de verrouillage 4 sous la tête 2, en engageant ses doigts de verrouillage 6 à travers la tête puis, avec la bague 11, on pousse le ressort 9 contre la pièce de verrouillage 4 et l'on comprime ce ressort en faisant simultanément tourner vers la gauche la bague 11 jusqu'à ce que son ergot 12 s'engage dans le cran de baïonnette 8, respectivement dans l'un des crans de baïonnette, dans lequel il vient s'accrocher en retenant la bague 11 poussée en arrière par le ressort 9. Le porte-outil peut alors être utilisé comme décrit dans le brevet EP 0 704 191. La partie évasée tronconique 10 donne prise au pouce vers l'index pour retirer en arrière la pièce de verrouillage 4 contre l'action du ressort 9 pour libérer l'outil fixé sur le porte-outil.

Inversement, pour démonter le porte-outil, il suffit de pousser en avant la bague 11 contre l'action du ressort 9 et de la faire tourner dans le sens des aiguilles d'une montre de telle manière que son ergot 12 soit poussé hors du cran de baïonnette 8 par le ressort 9.

On voit que le montage et le démontage du porte-outil se font instantanément et qu'ils peuvent se faire d'une seule main.

Tel que représenté à la figure 1, le manche 1 permet aux composants 4, 9 et 11 de s'échapper totalement du manche. Compte tenu du jeu important que les pièces 4 et 11 ont sur le manche 1, un tel démontage complet n'est pas nécessaire pour le nettoyage. Il est donc possible de prévoir une butée à l'extrémité du manche pour retenir les composants sur le manche.

L'extrémité du manche opposée à la tête 2 est représentée cylindrique mais elle peut présenter une autre forme, notamment une section hexagonale pour la fixation du porte-outil sur les moyens d'entraînement du porte-outil en rotation.

La bague 11 pourrait être rendue solidaire du manche par vissage c'est-à-dire en ayant un pas de vis dans la bague et sur la partie 7.

La tête 2 et les doigts 6 ne sont qu'un exemple de fixation d'outil parmi tous les moyens envisageables.

## Revendications

1. Porte-outil pour outil chirurgical comprenant:
un manche (1) muni d'une tête (2) conformée pour recevoir un outil et une pièce annulaire de verrouillage (4) montée coulissante le long du manche, sous la tête, munie de moyens de verrouillage (6) coopérant avec la tête pour verrouiller l'outil sur la tête et poussée contre la tête par un ressort hélicoïdal (9),
**caractérisé en ce que** le ressort de poussée (9) s'appuie sur une bague (11) capable de coulisser sur le manche et **en ce que** le manche (1) et la bague (11) présentent des moyens de solidarisation/ désolidarisation (8, 12) de telle manière que la libération de la bague autorise le libre coulissement de la pièce de verrouillage, du ressort et de la bague sur le manche.

2. Porte-outil selon la revendication 1, **caractérisé en ce que** le manche présente, sous la tête, une section (7) de diamètre supérieur au diamètre du reste du manche (1), section sur laquelle la bague (11) vient se fixer par une fixation à baïonnette (8, 12).

3. Porte-outil selon la revendication 1, dans lequel le manche (1) comprend en outre une section (7) située en dessous de la tête (2), ladite section (7) ayant un diamètre supérieur à celui du reste du manche (1).

4. Porte-outil selon la revendication 1, dans lequel la pièce annulaire de verrouillage (4) comprend une pluralité de doigts (6) s'étendant à travers la tête (2) pour former le verrouillage.

5. Porte-outil selon la revendication 1, dans lequel la bague (11) est couplée au manche (1) libre en coulissement.

6. Porte-outil selon la revendication 1, dans lequel la bague (11) est couplée au manche par vissage.

7. Porte-outil selon la revendication 1, dans lequel la bague (11) est fixée au manche par un ergot (12) pouvant se décrocher.

8. Porte-outil selon la revendication 1 , dans lequel la bague (11) est fixée au manche (1) par une fixation à baïonnette (8).

9. Porte-outil selon la revendication 1, dans lequel le ressort (9) est pré-comprimé entre la bague (11) et la pièce de verrouillage (4).

10. Porte-outil selon la revendication 1, dans lequel l'ergot (12) est placé sur la bague (11) et s'attache dans un fixation à baïonnette (8) située sur le manche (1), lorsque le bague (1) est tournée pour assurer l'assemblage.

## Claims

1. An instrument holder for a surgical instrument comprising a shank (1) equipped with a head (2) adapted to receive an instrument and an annular locking component (4) mounted so as to slide along the shank, under the head, the shank being equipped with locking means (6) which cooperate with the head so as to lock the instrument on the head, and the locking component (4) being pushed against the head by a helical spring (9), wherein the thrust spring (9) bears on a ring (11) capable of sliding along the shank, and wherein the shank (1) and the ring (11) have means of connection (8, 12) engaged by rotation of the ring, in such a way that the release of the ring allows the locking component, the spring and the ring to slide freely on the shank.

2. The instrument holder as claimed in claim 1, wherein the shank has, under the head, a section (7) with a diameter greater than the diameter of the rest of the shank (1), on which section the ring (11) is fixed by a bayonet fastening (8, 12).

3. The instrument holder as claimed in claim 1, wherein the shank (1) includes, among other things, a section (7) situated below the head (2), said section (7) having a diameter greater than the rest of the shank (1).

4. The instrument holder as claimed in claim 1, wherein the annular locking piece (4) includes a plurality of fingers (6) extending across the head (2) for forming the lock.

5. The instrument holder as claimed in claim 1, wherein the ring (11) is slidably coupled to the shank (1).

6. The instrument holder as claimed in claim 1, wherein the ring (11) is coupled to the shank by screwing.

7. The instrument holder as claimed in claim 1, wherein the ring (11) is fixed to the shank by a pin (12) permitting its removal.

8. The instrument holder as claimed in claim 1, wherein the ring (11) is fixed to the shank by a bayonet connection (8).

9. The instrument holder as claimed in claim 1, wherein the spring (9) is disposed compressed between the ring (11) and the locking piece (4).

10. The instrument holder as claimed in claim 1, wherein the pin (12) is placed on the ring (11) and attached via a bayonet fastening (8) disposed on the shank (1), then the ring (11) is turned to complete the assembly.

## Patentansprüche

1. Werkzeugträger für ein chirurgisches Werkzeug, der aufweist:
einen Stiel (1) mit einem Kopf (2), der angepasst ist, um ein Werkzeug aufzunehmen, und ein ringförmiges Verriegelungsteil (4), das entlang des Stiels unter dem Kopf gleitend montiert und
mit Verriegelungsmitteln (6) versehen ist, die mit dem Kopf zusammenwirken, um das Werkzeug auf dem Kopf zu verriegeln, und von einer Spiralfeder (9) gegen den Kopf gedrückt wird,
**dadurch gekennzeichnet, dass** die Schubfeder (9) sich auf einen Ring (11) stützt, der auf dem Stiel gleiten kann, und dass der Stiel (1) und der Ring (11) Mittel zur festen Verbindung/zum Lösen (8, 12) aufweisen, derart, dass die Freigabe des Rings das freie Gleiten des Verriegelungsteils, der Feder und des Rings auf dem Stiel erlaubt.

2. Werkzeugträger nach Anspruch 1, **dadurch gekennzeichnet, dass** der Stiel unter dem Kopf einen Abschnitt (7) mit einem Durchmesser aufweist, der größer ist als der Durchmesser des restlichen Stiels (1), ein Abschnitt, auf dem der Ring (11) durch eine Bajonettbefestigung (8, 12) befestigt wird.

3. Werkzeugträger nach Anspruch 1, bei dem der Stiel (1) außerdem einen Abschnitt (7) aufweist, der sich unter dem Kopf (2) befindet, wobei der Abschnitt (7) einen größeren Durchmesser als der Rest des Stiels (1) aufweist.

4. Werkzeugträger nach Anspruch 1, bei dem das ringförmige Verriegelungsteil (4) mehrere Finger (6) aufweist, die sich durch den Kopf (2) hindurch erstrecken, um die Verriegelung zu bilden.

5. Werkzeugträger nach Anspruch 1, bei dem der Ring (11) frei gleitend mit dem Stiel (1) gekoppelt ist.

6. Werkzeugträger nach Anspruch 1, bei dem der Ring (11) durch Schraubverbindung mit dem Stiel gekoppelt ist.

7. Werkzeugträger nach Anspruch 1, bei dem der Ring (11) durch einen Zapfen (12) am Stiel befestigt ist, der sich aushaken kann.

8. Werkzeugträger nach Anspruch 1, bei dem der Ring (11) durch eine Bajonettbefestigung (8) am Stiel (1) befestigt ist.

9. Werkzeugträger nach Anspruch 1, bei dem die Feder (9) zwischen dem Ring (11) und dem Verriegelungsteil (4) vorgespannt ist.

10. Werkzeugträger nach Anspruch 1, bei dem der Zapfen (12) auf dem Ring (11) angeordnet ist und sich in einer Bajonettbefestigung (8) befestigt, die auf dem Stiel (1) angeordnet ist, wenn der Ring (11) gedreht wird, um den Zusammenbau zu gewährleisten.
